# EUROPEAN PATENT APPLICATION

(11) **EP 4 079 330 A1**
(43) Date of publication of application: **26.10.2022**
(21) Application number: 21169934.3
(22) Date of filing: 22.04.2021
(51) Int. Cl.: A61K 49/00, B82Y 5/00, C12N 15/00

(54) **CONFORMATION SWITCHING NANOSTRUCTURES**

(71) Applicant: Technische Universität München, 80333 München (DE)
(72) Inventor: Engelen, Wouter, 80798 München (DE); Sigl, Christian, 82327 Tutzing (DE); Dietz, Hendrik, 85540 Haar (DE)
(74) Representative: Engelhard, Markus

(57) **Abstract**

The present invention relates to conditionally switchable nanostructures, and systems comprising the same. In particular, the present invention pertains to a method for linking the identification of at least one molecule to a conformational reconfiguration of a nanostructure. The invention is further based on the finding that coupling molecules, such as antibodies or antigen binding fragments, can stabilize subunit interactions of nanostructures at otherwise unfavourable conditions. Additionally, the present invention also relates to a system comprising the nanostructures of this invention as well as methods for utilizing the nanostructures or the system of this invention. Finally, substances and/or compositions comprising at least one or a plurality of nanostructures for use as a medicament, or for use in a diagnostic method, are also provided.

## Description

### FIELD OF THE INVENTION

The present invention relates to conditionally switchable nanostructures, and systems comprising the same. In particular, the present invention pertains to a method for linking the identification of at least one molecule to a conformational reconfiguration of a nanostructure. The invention is further based on the finding that coupling molecules, such as antibodies or antigen binding fragments, can stabilize subunit interactions of nanostructures at otherwise unfavourable conditions. Additionally, the present invention also relates to a system comprising the nanostructures of this invention as well as methods for utilizing the nanostructures or the system of this invention. Finally, substances and/or compositions comprising at least one or a plurality of nanostructures for use as a medicament, or for use in a diagnostic method, are also provided.

### DESCRIPTION

Molecular recognition is a fundamental feature of living systems and is required for e.g. T-cell recognition by the adaptive immune system or transduction of molecular signals across the cellular membranes. A key advancement in modern medicine is the development of therapeutics that demonstrate similar molecular recognition characteristics, such as therapeutic antibodies targeting specific cell surface receptors. Recent advancements in the field of DNA nanotechnology have rendered it a potential candidate for the development of "intelligent" nanoscale devices that show similar molecular recognition characteristics as those observed in naturally occurring molecules. To develop such intelligent nanostructures, molecular recognition must be coupled to signal transduction mechanisms, conferring conformational reconfigurations typically but not exclusively resulting in the release of cargo from drug carriers or the activation of therapeutic agents.

In recent years, new methods for manufacturing nanostructures have been developed. These include methods for manufacturing nucleic acid nanostructures, as disclosed in US 7,842,793 B3. The disclosure relates to a technique referred to as "DNA origami", which is a method for generating nanoscale devices, systems, and enzyme factories based upon nucleic acid nanostructures that can be designed to have a predetermined structure. DNA origami uses DNA molecules for the production of three dimensional structures on the nanometer scale. Chemical modifications or single-stranded DNA overhangs can be placed on origami structures to design and build the desired structure of the nucleic acid nanostructures, and to link the DNA origami structures to other molecules. DNA origami structures can, therefore, be used to modulate cellular signaling mechanisms.

Recently, DNA origami techniques have been used for the manufacture of different nanoscale devices. As an example, WO 2012/061719 A2 discloses DNA origami devices useful in the targeted delivery of biologically active entities to specific cell populations. While this device may enable a targeted delivery of biologically active entities, it relies on specific interaction molecules, in particular on aptamers, that may bind to two different binding targets.

US 9,863,930 B2 discloses various molecular barcoded bi-stable switches that can be used to detect different analytes. In particular, the molecular barcoded bi-stable switch may be manufactured using DNA origami.

However, integration of modules that control reconfiguration of nanoparticles induced by molecular signals remains a largely unmet challenge. To date, most of the few developed DNA-origami nanoparticles that show some sort of molecular reconfiguration rely on structure switching aptamers. Typically, these aptamers lock the structure in a meta-stable closed configuration. Binding of the antigen to the aptamer locks induce an equilibrium shift to the open state. An inherent limitation of the use of structure switching aptamers is this intricate balance between the stability of open and closed states, which requires extensive tuning for different structures and aptamer types. In addition, only a limited number of aptamers are known for an even smaller number of antigens and they typically bind with moderate affinities, or require exotic metal ions for proper functioning. Finally, aptamers show a large structural heterogeneity, which makes modular design inherently difficult.

In view of the above, there is a continuing need in the art to develop nanostructures that allow a molecular recognition to be coupled to signal transduction mechanisms. There is also an urgent need for the development of nanostructures that can recognize a large number of molecules, and reliably change their conformation upon recognition. Moreover, nanostructures that bind to target molecules with high affinities are also urgently needed. It is an object of the present invention to overcome or at least alleviate the shortcomings and disadvantages of the prior art. That is, it is an object of the present invention to provide novel conformation switching nanostructures.

### BRIEF DESCRIPTION OF THE INVENTION

Generally, and by way of brief description, the main aspects of the present invention can be described as follows:
In **a first aspect,** the invention pertains to a nanostructure comprising at least two coupling sites.
In **a second aspect,** the invention pertains to a system comprising a nanostructure.
In **a third aspect,** the invention pertains to a method for linking an identification of at least one molecule to a conformational reconfiguration of a nanostructure.
In **a fourth aspect,** the invention pertains to a substance comprising at least one or a plurality of nanostructures for use as a medicament, or for use in a diagnostic method.
In **a fifth aspect,** the invention pertains to a composition comprising at least one or a plurality of nanostructures for use as a medicament, or for use in a diagnostic method.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In the first aspect, the invention pertains to a nanostructure comprising at least two coupling sites, wherein said at least two coupling sites may bind to at least one coupling molecule (i.e., to one coupling molecule or to a plurality of coupling molecules), and wherein said at least two coupling sites are configured to be a coupling site set.

In a preferred embodiment, the coupling site set is configured to bind to one coupling molecule. Generally, it should be understood that the nanostructure comprising at least two coupling sites should not be construed to mean that the nanostructure only comprises these two coupling sites, and that the one coupling site set is configured to bind to only one coupling molecule. Instead, the nanostructure may also have more than two coupling sites, and one coupling site set may be configured to bind to more than one coupling molecule.

In another preferred embodiment, the nanostructure comprises at least one first entity and at least one second entity, wherein each of the at least one first entity and the at least one second entity comprises at least one coupling site of at least one coupling site set, preferably wherein the at least one coupling site of the at least one first entity and the at least one coupling site of the at least one second entity are located on symmetrical locations of the at least one first and of the at least one second entity.

In a preferred embodiment, the coupling site set may comprise only identical coupling sites, i.e. coupling sites configured to couple to the same coupling molecules. In other embodiments, the coupling site set may comprise at least two distinct coupling sites, configured to couple to a coupling molecule by different interactions. The coupling site set may be formed by two coupling sites. However, in other embodiments, the coupling site set may also be formed by a plurality of coupling sites.

This invention generally relates to nanostructures. In examples of the present invention, the nanostructure may be a DNA origami structure, i.e., a DNA origami device, as exemplarily disclosed in US 7,842,793 B2. However, it should be understood that the present technology is not limited thereto, and it should be understood that this is merely exemplary and that also other nanostructures may be utilized to exercise the present invention. However, as used herein, the term "nanostructure" shall preferably refer to a DNA-origami structure, which is composed of multiple DNA-origami subunits (entities). In some embodiments, the inside of the nanostructure according to this invention can be hollow and comprise a molecule, such as a therapeutic molecule and/or a cargo molecule.

The term "entity" shall refer to a nanostructure and/or DNA-origami subunit.

The term "coupling site" shall refer to a site on a subunit ("entity") of the nanostructure, to which a coupling molecule binds. Preferably, a coupling site is an antigen, an antigenic fragment, or an antigenic mimetic. Alternatively, the coupling site is a biotin molecule, a streptavidin molecule, or any other molecule specifically binding to a particular different molecule.

A "coupling site set", as used herein, shall refer to a coupling site set that consists of at least, preferably of, two coupling sites. Preferably, the coupling molecule is configured to couple one subunit (e.g., a "first entity") to a second subunit (e.g., a "second entity") of the nanostructure when binding to one coupling site set (e.g., by binding of an antibody to two antigen binding constructs on different DNA-origami subunits).

The term "at least one" according to the present invention shall include 1, 2, 3, 4, 5, 6, 7, 8, 9, 10, 11, 12, 13, 14, 15, 16, 17, 18, 19, 20, or any other number. For example, the term "at least one" first entity shall include 1, 2, 3, 4, 5, 6, 7, 8, 9, 10, 11, 12, 13, 14, 15, 16, 17, 18, 19, 20, or any other number of first entities. Similarly, the term "at least one" second entity shall include 1, 2, 3, 4, 5, 6, 7, 8, 9, 10, 11, 12, 13, 14, 15, 16, 17, 18, 19, 20, or any other number of second entities.

In yet another embodiment, the at least one coupling molecule is configured to couple the at least one first entity of the nanostructure to the at least one second entity of the nanostructure when the at least one coupling molecule is binding to the at least one coupling site set.

The term "coupling molecule" shall refer to a molecule binding to a coupling site. Preferably, a coupling molecule is an antigen binding construct, such as an antibody. Alternatively, the coupling site is a streptavidin molecule or any other molecule specifically binding to a different molecule. In the main assay of this invention, the coupling molecule is artificially added to the assay. The coupling molecule may bind reversibly to the coupling site, i.e. the coupling molecule may bind reversibly to the coupling site and detach repeatedly. Accordingly, a coupling molecule can couple and decouple repeatedly to a coupling site.

Also preferred is that the nanostructure comprises a plurality of the at least one first entity and of the at least one second entity, such as at least two, three, four, five, six, seven, eight, nine, ten, eleven, twelve, thirteen, fourteen, fifteen, sixteen, seventeen, eighteen, nineteen, twenty, twenty one, twenty two, twenty three, twenty four, twenty five, twenty six, twenty seven, twenty eight, twenty nine, thirty, or any number of the at least one first entity, and at least one, two, three, four, five, six, seven, eight, nine, ten, eleven, twelve, thirteen, fourteen, fifteen, sixteen, seventeen, eighteen, nineteen, twenty, twenty one, twenty two, twenty three, twenty four, twenty five, twenty six, twenty seven, twenty eight, twenty nine, thirty, or any number of the at least one second entity.

A further embodiment relates to the nanostructure of this invention, wherein the nanostructure comprises a plurality of coupling site sets, preferably at least two, three, four, five, six, seven, eight, nine, ten, eleven, twelve, thirteen, fourteen, fifteen, sixteen, seventeen, eighteen, nineteen, twenty, twenty one, twenty two, twenty three, twenty four, twenty five, twenty six, twenty seven, twenty eight, twenty nine, thirty, or any number of coupling site sets, preferably wherein each coupling site set consists of two coupling sites.

Yet another embodiment relates to the nanostructure of this invention, wherein the coupling molecule binding to a first coupling site set of the plurality of coupling site sets is the same coupling molecule as the coupling molecule binding to a second coupling site set of the plurality of coupling site sets, or wherein the coupling molecule binding to the first coupling site set of the plurality of the coupling site sets is a different coupling molecule than the coupling molecule binding to the second coupling site set of the plurality of coupling site sets.

The nanostructure may be configured to assume different configurations including at least one first configuration (A), and at least one second configuration (B). In some embodiments, these configurations may also be referred to as an "open" and "closed" configuration. Depending on the configuration the nanostructure assumes, the nanostructure may change the accessibility of different parts of the nanostructure. For example, the interior of the nanostructure may be more accessible in the second configuration (B) than in the first configuration (A).

In a preferred embodiment, the nanostructure is configured to assume at least one first configuration (A), and at least one second configuration (B), optionally at least one third configuration (C), at least one fourth configuration (D), or any other number of configurations. Thus, the nanostructure of any of the embodiments of this invention can assume different configurations (e.g. configuration (A), (B), (C), (D), etc.).

Depending on whether or not the coupling sites are coupled to coupling molecules, a likelihood of the nanostructure assuming the different configurations may change. As an example, when all (or a part) of the coupling sites are coupled by coupling molecules, the nanostructure may be more likely to assume, e.g., the first configuration (A) than would be the case when none of the coupling sites are coupled by coupling molecules. Thus, depending on the coupling state of the coupling sites, the accessibility of the interior of the nanostructure may be changed. Therefore, the coupling state of the coupling sites may "open" and/or "close" the nanostructure. It will be understood that this may also include the release of molecules, such as cargo molecules and/or therapeutic molecules that may be present in the interior of the nanostructure.

The nanostructure may be configured to assume the first configuration (A) with a higher probability than the second configuration (B) when at least one coupling molecule is binding to the at least one respective coupling site set. A particularly preferred embodiment, therefore, relates to the nanostructure of this invention, wherein the nanostructure is configured to assume the at least one first configuration (A) when at least one coupling molecule is binding to at least one coupling site set, and/or wherein the nanostructure is configured to assume the at least one second configuration (B) when at least one coupling molecule is not binding to at least one coupling site set.

However, also when no coupling molecule is binding to a respective coupling site set, the nanostructure may assume both the first configuration (A) and the second configuration (B). More particularly, there may be an equilibrium between the first configuration (A) and the second configuration (B). In this equilibrium, the first configuration (A) may be "preferred", i.e., the nanostructure may assume this first configuration (A) with a higher likelihood than the second configuration (B), or vice versa. Further, the nanostructure maybe configured to assume at least one or a plurality of equilibrium states between the first configuration (A) and the second configuration (B). For example, the nanostructure may be configured to assume a first and a second equilibrium state between the first configuration (A) and the second configuration (B), wherein the probability that the nanostructure assumes the second configuration (B) may be different (e.g., higher) in the second equilibrium state than in the first equilibrium state.

It will be understood that the above is merely an example and that the first and second equilibrium state may assume the first configuration (A) and the second configuration (B) with different probabilities. In general, the second configuration (B) may be assumed with a higher probability in the second equilibrium state than in the first equilibrium state.

In another embodiment, the nanostructure is configured to assume the at least one first configuration (A) when little or no amount of at least one competing molecule is present, and/or wherein the nanostructure is configured to assume the at least one second configuration (B) when a higher amount of at least one competing molecule is present.

Thus, when the nanostructure is in an environment where little or no amount of the at least one competing molecule is present, it is more likely that such a nanostructure has configuration (A). Contrary, when the nanostructure is in an environment where a higher amount of the at least one competing molecule is present, it is more likely that such a nanostructure has configuration (B). Generally, a nanostructure may assume the first configuration (A) when each coupling site, or when a subset of the coupling site sets, is coupled to its respective coupling molecule. At least one, or a multitude of competing molecules, can, however, induce and/or trigger the release of at least one coupling molecule. Hence, when the nanostructure is in an environment where a high amount of the at least one competing molecule is present, it is more likely that at least one coupling molecule is released from the nanostructure. In such an event, the nanostructure can reconfigure from, e.g. configuration (A) to configuration (B).

The term "competing molecule", as used herein, refers to a molecule that is preferably an antigen, an antigenic fragment, or an antigenic mimetic. Alternatively, the competing molecule may be a biotin molecule, a streptavidin molecule, or any other molecule specifically binding to a particular different molecule. The competing molecule is competing with the coupling site for binding the at least one coupling molecule. The competing molecule may be present in, *e.g.,* a sample, such as a body liquid sample, a cell, an organism, or the like. It is one aim of this invention to detect a competing molecule.

Generally, it will be understood that the nanostructure can also assume the configuration (A) (e.g., the closed configuration) and/or configuration (B) (e.g., the open configuration) when the coupling sites are not, are partially, or are all coupled to their respective coupling molecules.

Yet another preferred embodiment relates to the nanostructure of this invention, wherein the nanostructure is configured to assume the at least one first configuration (A) in a first solution, wherein the first solution preferably comprises a higher concentration of magnesium ions compared to a second solution, and/or wherein the nanostructure is configured to assume the at least one second configuration (B) in the second solution, wherein the second solution preferably comprises a lower concentration of magnesium ions compared to the first solution.

However, it shall be understood that the conditions of the first and the second solution can vary, depending on the experimental and/or biological setup. The person of skill is aware how to determine ideal conditions of the first and the second solution. For example, in one embodiment, the magnesium ion concentration of the first solution is higher than 20 mM, and/or the magnesium ion concentration of the second solution is lower than 20 mM.

In some embodiments, the nanostructure may comprise a first portion and a second portion, wherein the first portion may be movable with respect to the second portion. Moreover, the first portion and the second portion may each comprise one of the at least two coupling sites of a coupling site set that may bind to the at least one coupling molecule, or the first portion and the second portion may each comprise at least two coupling sites that may bind to at least one coupling molecule. In some preferred embodiments, a "portion" may be an entity.

Further, a subset of a coupling site set may comprise at least one coupling site comprised by the first portion and at least one coupling site comprised by the second portion. That is, each portion may comprise at least one coupling site. The first portion and the second portion may be movably attached to each other, e.g. by means of a hinge or a rotational axis.

Generally, it should be understood that the nanostructure comprising, e.g. a first portion and a second portion, should not be construed to mean that the nanostructure only comprises one first portion and one second portion. Instead, the nanostructure may also have more than one first portion and more than one second portion. In other words (unless explicitly stated or unless clear to the skilled person), usage of an article (e.g., "a", "an") should not be understood to exclude the plural. For example, also a nanostructure comprising two first portions and/or two second portions is to be construed to be encompassed by "a nanostructure comprising a first portion and a second portion". In some embodiments, the nanostructure may comprise at least one additional portion, i.e. a third, a fourth, a fifth, a sixth, a seventh, an eighth, or any other number of an additional portion.

Particularly preferred is that the nanostructure comprises at least one molecule selected from the group consisting of a nucleic acid, such as a DNA, an RNA, a DNA aptamer, an RNA aptamer, or a peptide nucleic acid (PNA), a polypeptide, a peptide, a glycoprotein, a peptidomimetic, and an antigen binding construct, such as an antibody, an antibody-like molecule, an antigen binding derivative, or an antigen binding fragment thereof.

In a further preferred embodiment, the nanostructure comprises at least one nucleic acid, wherein said nucleic acid may comprise at least one modification, for example a chemical modification selected from a modified internucleoside linkage, a modified nucleobase, or a modified sugar moiety, such as a 2'-O-alkyl modification, for example a 2'-O-methoxy-ethyl (MOE) or 2'-O-Methyl (OMe) modification, an ethylene-bridged nucleic acid (ENA), a 2'-fluoro (2'-F) nucleic acid, such as 2'-fluoro N3-P5'-phosphoramidite, a 1',5'-anhydrohexitol nucleic acid (HNA), or a locked nucleic acid (LNA).

Yet another embodiment relates to the nanostructure of this invention, wherein the nanostructure is at least partially formed by a DNA origami structure. The DNA origami structure may comprise at least one scaffolding strand, i.e. preferably single-stranded polynucleotide scaffold DNA with a known sequence, and optionally at least one staple strand. As used herein, the term "DNA-origami structure" shall refer to a nanostructure comprising at least one scaffolding strand and a plurality of single-stranded oligonucleotide staple strands. The DNA origami structures may form by self-assembly and available software for designing the corresponding scaffolding and staple strands may assist in the DNA-origami manufacturing technique.

The term "scaffolding strand", as used herein, shall refer to a strand that makes up and/or traverses the main part of a DNA-origami structure and/or the DNA-origami subunits ("entities").

The term "staple strand", as used in this invention, shall refer to a single-stranded oligonucleotide molecule, which is at least partially complementary to a scaffolding strand. In general, staple strands can be used to introduce, e.g., coupling sites into a DNA-origami structure and/or the DNA-origami subunit ("entity").

The DNA origami structure may comprise a plurality of single-stranded oligonucleotide staple strands, wherein each staple strand may be at least partially complementary to at least one scaffolding strand. Further, each of the staple strands may be configured to bind to the at least one scaffolding strand in two distinct places, wherein the at least one scaffolding strand may be folded and/or arranged such that the desired nanostructure may be formed.

A preferred embodiment relates to the nanostructure of this invention, wherein the nanostructure is at least partially formed by a DNA origami structure,
wherein the DNA origami structure comprises at least one scaffolding strand;
wherein the DNA origami structure further comprises a plurality of single-stranded oligonucleotide staple strands;
wherein each staple strand is at least partially complementary to at least one scaffolding strand; and
wherein each of the staple strands is configured to bind to at least one of the at least one scaffolding strands, wherein the at least one scaffolding strand is folded and/or arranged such that the desired nanostructure is formed.

In a further embodiment, at least one coupling site is present in at least one staple strand.

Moreover, in embodiments where the nanostructure is at least partially formed by a DNA origami structure and where the DNA origami structure comprises at least one scaffolding strand and a plurality of staple strands, the molecule may be bound to one of the at least one scaffolding strands or a staple strand by means of a linker molecule, wherein the linker molecule may be connected to a DNA strand portion, which is complementary to a portion of the at least one scaffolding strand or to a portion of a staple strand.

In those embodiments, where the nanostructure comprises at least one first entity and at least one second entity, the at least one first entity and the at least one second entity may comprise an identical shape, or a distinguishable shape. Further, the shape maybe, for example, a triangular shape, a round shape, a cuboid, i.e. a rectangular shape, or any other shape. Similarly, in embodiments where the nanostructure comprises a first and second portion, the first portion and the second portion may comprise an identical shape, or a distinguishable shape. The shape may be, for example, a triangular shape, a round shape, a cuboid, i.e. a rectangular shape, or any other shape.

In some embodiments, the at least one coupling site may be a molecule. A preferred embodiment relates to the nanostructure of this invention, wherein the at least one coupling site is a molecule. In a particularly preferred embodiment, the at least one coupling site, and/or the at least one coupling molecule is a molecule selected from the group consisting of an antigen, an antigenic fragment, an antigenic mimetic, an antigen binding construct, such as an antibody, an antibody-like molecule, an antibody mimetic, an antigen binding derivative, or an antigen binding fragment, a DNA strand, a biotin molecule, a streptavidin molecule, a maleimide-thiol chemistry molecule, a click chemistry molecule, a SNAP-tag protein, and a CLIP-tag molecule, or the like.

In a particularly preferred embodiment, the coupling site is an antigen, an antigenic fragment, and/or an antigenic mimetic.

In a further embodiment, the coupling molecule is an antigen binding construct, such as an antibody, an antibody-like molecule, an antibody mimetic, an antigen binding derivative, or an antigen binding fragment thereof.

Accordingly, in one preferred embodiment, the interaction between coupling site and coupling molecule is characterized by the specific binding of an antibody-antigen interaction. The person of skill is able to identify similar specific interactions, which shall also be within the scope of this invention. As an example, the specific binding of a streptavidin molecule to a biotin molecule may also be used, e.g. the coupling site may be a streptavidin molecule and the coupling molecule may be a biotin molecule, or vice versa. However, a great variety of interactions may be utilized in such a nanostructure, which may be advantageous compared to the state of the art that relies on specific and limited interactions. Thus, owing to the wide range of interactions available, a more versatile and flexible conditionally switchable nanostructure is provided compared to nanostructures disclosed in the prior art.

Also preferred is that the coupling molecule is an IgG antibody, an IgG antibody fragment, an IgG antibody mimetic, an IgM antibody, an IgM antibody fragment, an IgM antibody mimetic, an IgD antibody, an IgD antibody fragment, an IgD antibody mimetic, an IgA antibody, an IgA antibody fragment, an IgA antibody mimetic, an IgE antibody, an IgE antibody fragment, or an IgE antibody mimetic, preferably wherein said antibody, antibody fragment, or antibody mimetic comprises two identical antigen binding sites, more preferably wherein said antigen binding sites bind to said at least two coupling sites of a coupling site set.

Yet another particularly preferred embodiment relates to the above nanostructure, wherein one IgG antibody, one IgG antibody fragment, one IgG antibody mimetic, one IgM antibody, one IgM antibody fragment, one IgM antibody mimetic, one IgD antibody, one IgD antibody fragment, one IgD antibody mimetic, one IgA antibody, one IgA antibody fragment, one IgA antibody mimetic, one IgE antibody, one IgE antibody fragment, or one IgE antibody mimetic is binding to one coupling site set.

The nanostructure of this invention can be of any length. In a preferred embodiment, the nanostructure comprises a maximum length, and in a particularly preferred embodiment, the maximum length is smaller than 1000 nm, preferably smaller than 500 nm, such as around 100 nm, or smaller.

A further aspect of this invention relates to a system comprising the nanostructure according to this invention, wherein the system comprises
(i) at least one nanostructure comprising at least two coupling sites, wherein said at least two coupling sites may bind to at least one coupling molecule, and wherein said at least two coupling sites are configured to be a coupling site set, and
(ii) at least one coupling molecule.

The term "system" shall refer to a structure comprising at least one DNA-origami structure ("nanostructure") and at least one coupling molecule. In some embodiments, the system may, however, compromise a plurality of coupling molecules.

In a preferred embodiment, the system comprises
(i) at least one nanostructure comprising at least one first entity and at least one second entity, wherein each of the at least one first entity and the at least one second entity comprises at least one coupling site of at least one coupling site set, preferably wherein the at least one coupling site on the at least one first entity and the at least one coupling site on the at least one second entity are located on symmetrical locations of the entities, and
(ii) at least one coupling molecule, preferably a plurality of coupling molecules,
wherein the at least one coupling molecule is configured to couple the at least one first entity of the nanostructure to the at least one second entity of the nanostructure when the at least one coupling molecule is binding to the at least one coupling site set.

Yet another aspect of this invention, which can be combined with any of the other preferred embodiments and/or aspects of the invention, pertains to a method for conditionally switching between different configurations of the nanostructure of the present invention, thereby enabling to link the identification of at least one molecule to a conformational reconfiguration of a nanostructure. The latter may, in a specific embodiment, allow the release of a therapeutic and/or a cargo molecule, which can be comprised in a cavity and/or a hole within the nanostructure of the present invention. Therefore, the method can allow the nanostructure to specifically release a molecule upon identification of a particular molecule.

In other words, the present invention relates to a conditionally switchable nanostructure that may assume different configurations depending on the coupling of at least a subset of the coupling site set comprised by the nanostructure, wherein the accessibility of a therapeutic and/or a cargo molecule may change depending on the configuration the nanostructure assumes. For example, a therapeutic and/or a cargo molecule may be significantly more accessible in one configuration than the other, in some cases it may even be only accessible in one of the two configurations. This may allow enabling access to the therapeutic and/or cargo molecule conditioned on the coupling of at least a subset of coupling sites of the coupling site sets. This may be advantageous as it may control the release of the molecule, such as the therapeutic and/or cargo molecule, based on the presence or absence of at least a subset of the respective competing molecules.

The term "identification of a molecule" shall relate to the identification of, e.g., an antigen, a diseased cell, such as a tumor cell, and the like. Said identification can occur *in-vivo, ex-vivo,* or *in-vitro.* In a preferred embodiment, the identification occurs *in-vivo* in an organism, such as a mammal, preferably a human. In another embodiment, the identification occurs *in-vitro* in a sample obtained from, e.g. an organism, such as a mammal, preferably a human. The nanostructure is preferably assuming a different configuration upon identifying a particular molecule, such as the competing molecule, thereby linking the identification of the molecule, such as the competing molecule, to a conformational reconfiguration of the nanostructure. Further included within the scope of this invention is the necessity to identify an amount of particular molecules, such as competing molecules, that is higher than or corresponds to a particular threshold of the particular molecules, such as competing molecules, for inducing the conformational reconfiguration of the nanostructure.

The term "conformational reconfiguration" refers to the reconfiguration from one configuration to another one, e.g. from configuration (A) to configuration (B). The nanostructure is preferably assuming configuration (B) when at least one competing molecule is competing with at least one coupling molecule, and the at least one coupling molecule is not binding to at least one coupling site set. Upon a conformational reconfiguration, a molecule, such as a therapeutic molecule and/or a cargo molecule, which is comprised within the hollow inner part of the nanostructure, can be released and/or activated.

A preferred aspect of this invention, which can be combined with any of the other preferred embodiments and/or aspects of the invention, pertains to a method for linking an identification of at least one molecule to a conformational reconfiguration of a nanostructure, wherein the method comprises:
(i) providing a nanostructure according to this invention;
(ii) providing at least one coupling molecule,
   wherein the nanostructure is assuming at least one first configuration (A) when at least one coupling molecule as provided in (ii) is binding to at least one coupling site set of the nanostructure provided in (i), and
(iii) identifying the at least one molecule,
wherein said at least one molecule is preferably a competing molecule,
wherein the nanostructure is assuming the at least one second configuration (B) upon identifying the at least one molecule,
thereby enabling linking the identification of at least one molecule to a conformational reconfiguration of the nanostructure.

Whenever method steps are recited in this description and also in the appended claims, it should be noted that the order in which the steps are recited in this text may be accidental. That is, unless otherwise specified or unless clear to the skilled person, the order in which steps are recited may also differ. For example, when the present document states that, e.g., a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) can encompass the situation that step (X) is performed directly before step (Z), but also the situation that step (X) is performed before one or more steps, e.g. (Y1), (Y2), (Y3), followed by step (Z). Corresponding considerations apply when terms like "after" or "before" are used.

In a preferred embodiment of the method for linking an identification of at least one molecule to a conformational reconfiguration of a nanostructure, the nanostructure encloses a molecule or a second nanostructure in the at least one first configuration (A), preferably wherein the molecule is selected from a therapeutic molecule and/or a cargo molecule.

The different entities of the nanostructure may be configured to form a chamber and/or a cavity when the nanostructure is, *e.g.,* in the first configuration (A). Further, a therapeutic molecule and/or cargo molecule may be attached to an inner surface of the different entities of the nanostructure, for example by means of a rod, by means of a direct or indirect association, or by steric means. The therapeutic molecule and/or cargo molecule may be configured to leave the chamber and/or the cavity when the nanostructure is in the second configuration (B). The method may comprise coupling the coupling sites to identical coupling molecules. Alternatively, the method may comprise coupling the coupling sites to distinct coupling molecules.

Also preferred is that the molecule or second nanostructure, preferably the molecule selected from a therapeutic molecule and/or cargo molecule, is released and/or activated upon a conformational reconfiguration of the nanostructure when the nanostructure is assuming the at least one second configuration (B).

Accordingly, depending on the binding of coupling molecules to the coupling sites on the nanostructure, in simplified words, the nanostructure may be switched between different states. This allows many different applications. In particular, such a nanostructure can be used to carry therapeutic molecules and/or cargo molecules. This may, for example, be used for the controlled release of therapeutic and/or cargo molecules upon identification of, for example, a particular cell type and/or molecule. Hence, the therapeutic molecule and/or cargo molecule may be accessible in one configuration of the nanostructure, but not in a different configuration, and the configuration may differ depending on the presence or absence of a particular cell type and/or molecule. This may be particularly beneficial for applications where the accessibility to a therapeutic molecule and/or a cargo molecule should be conditioned on the presence or absence of a particular cell type and/or molecule, which is preferably achieved by a conformational reconfiguration of the nanostructure of this invention.

The different entities of the nanostructure may each comprise a cavity, configured to form a chamber and/or a hole when the nanostructure is, *e.g.,* in the first configuration (A). Further, the therapeutic molecule and/or cargo molecule may be attached to an inner surface of the different entities of the nanostructure, for example by means of a rod, by means of a direct or indirect association, or by steric means, wherein the therapeutic molecule and/or cargo molecule may be configured to leave the cavity when the nanostructure is in the second configuration (B). The person skilled in the art will appreciate that a rod may be any type of a flexible link between an entity and at least one therapeutic and/or cargo molecule.

In a preferred embodiment the method is an *in-vivo, ex-vivo, in-vitro,* or *in-situ* method.

A further embodiment relates to the method for linking an identification of at least one molecule to a conformational reconfiguration of a nanostructure, wherein the nanostructure is assuming the at least one second configuration (B) when the at least one molecule is competing with the at least one coupling molecule, and the at least one coupling molecule is not binding to at least one coupling site set, preferably wherein the magnesium ion concentration of the solution is lower than 20 mM.

In a further aspect, the present invention relates to a substance comprising a plurality of nanostructures according to the present invention, or comprising a system as defined above.

Another aspect of the present invention pertains to a composition comprising at least one or a plurality of nanostructures according to this invention, or comprising a system as defined above.

A further aspect of this invention, which can be combined with any of the other preferred embodiments and/or aspects of the invention, pertains to a substance comprising at least one or a plurality of nanostructures, or a system as defined above, for use as a medicament.

Yet another aspect of this invention, which can be combined with any of the other preferred embodiments and/or aspects of the invention, pertains to a composition comprising at least one or a plurality of nanostructures according to this invention, or a system as defined above, for use as a medicament.

The substance and/or the composition for use as a medicament may be advantageous, as the nanostructures of this invention may enable the targeted treatment of diseases, such as targeted drug delivery.

Further, the substance comprising at least one or a plurality of nanostructures according to this invention, or comprising a system as defined above, may be for use in the prevention and/or treatment of a proliferative disease, such as cancer, in the prevention and/or treatment of an immunological disorder, in the prevention and/or treatment of a viral disease, such as a human immunodeficiency virus (HIV) infection, in the prevention and/or treatment of a blood clotting disorder, in the prevention and/or treatment of a metabolic disorder, in the prevention and/or treatment of an infectious disorder, and/or in the prevention and/or treatment of diabetes.

In an additionally preferred aspect of this invention, the composition comprising at least one or a plurality of nanostructures according to this invention, or comprising a system as defined above, may be for use in the prevention and/or treatment of a proliferative disease, such as cancer, in the prevention and/or treatment of an immunological disorder, in the prevention and/or treatment of a viral disease, such as a human immunodeficiency virus (HIV) infection, in the prevention and/or treatment of a blood clotting disorder, in the prevention and/or treatment of a metabolic disorder, in the prevention and/or treatment of an infectious disorder, and/or in the prevention and/or treatment of diabetes.

A further aspect of this invention, which can be combined with any of the other preferred embodiments and/or aspects of the invention, relates to a substance comprising at least one or a plurality of nanostructures, or a system as defined above, for use in a diagnostic method.

Yet another aspect of this invention, which can be combined with any of the other preferred embodiments and/or aspects of the invention, pertains to a composition comprising at least one or a plurality of nanostructures according to this invention, or a system as defined above, for use in a diagnostic method.

Another aspect of this invention, which can be combined with any of the other preferred embodiments and/or aspects of the invention, pertains to a vector, in particular an expression vector, comprising a sequence coding for a nanostructure, a sequence of a scaffolding strand and/or a staple strand, and/or comprising the sequence of the nanostructure, as defined above.

A "vector" may be any agent that is able to deliver or maintain a nucleic acid in a host cell and includes, for example, but is not limited to, plasmids (e.g., DNA plasmids), naked nucleic acids, viral vectors, viruses, nucleic acids complexed with one or more polypeptide or other molecules, as well as nucleic acids immobilized onto solid phase particles. Vectors are described in detail below. A vector can be useful as an agent for delivering or maintaining an exogenous gene and/or protein in a host cell. A vector may be capable of transducing, transfecting, or transforming a cell, thereby causing the cell to replicate or express nucleic acids and/or proteins other than those native to the cell or in a manner not native to the cell. The target cell may be a cell maintained under cell culture conditions or in other in *vivo* embodiments, being part of a living organism. A vector may include materials to aid in achieving entry of a nucleic acid into the cell, such as a viral particle, liposome, protein coating, or the like. Any method of transferring a nucleic acid into the cell may be used; unless otherwise indicated, the term vector does not imply any particular method of delivering a nucleic acid into a cell or imply that any particular cell type is the subject of transduction. The present invention is not limited to any specific vector for delivery or maintenance of any nucleic acid of the invention, including, e.g., a nucleic acid encoding a nanostructure of this invention.

The term "expression vector" means any double-stranded DNA or double-stranded RNA designed to transcribe an RNA, e.g., a construct that contains at least one promoter operably linked to a downstream gene or coding region of interest (e.g., a cDNA or genomic DNA fragment that encodes a protein, or any RNA of interest). Transfection or transformation of the expression construct into a recipient cell allows the cell to express RNA or protein encoded by the expression construct. An expression construct may be a genetically engineered plasmid, virus, or an artificial chromosome derived from, for example, a bacteriophage, adenovirus, retrovirus, poxvirus, or herpesvirus, or further embodiments described under "expression vector" below. An expression construct can be replicated in a living cell, or it can be made synthetically. For purposes of this application, the terms "expression construct", "expression vector", "vector", and "plasmid" are used interchangeably to demonstrate the application of the invention in a general, illustrative sense, and are not intended to limit the invention to a particular type of expression construct. Further, the term expression construct or vector is intended to also include instances wherein the cell utilized for the assay already endogenously comprises such DNA sequence. An expression vector preferably comprises a promoter sequence operably linked to a nucleic acid.

The term "encoding" or more simply "coding" refers to the ability of a nucleotide sequence to code for one or more amino acids. The term does not require a start or stop codon. An amino acid sequence can be encoded in any one of six different reading frames provided by a polynucleotide sequence and its complement. An amino acid sequence can be encoded by desoxyribonucleic acid (DNA), ribonucleic acid (RNA), or artificially synthesized polymers similar to DNA or RNA.

In another aspect there is also provided a recombinant cell comprising a nanostructure and/or a vector as defined above. A "recombinant cell", sometimes also referred to as "host cell", is any cell that is susceptible to transformation with a nucleic acid. Preferably, the recombinant or host cell of the invention is a plant cell, a bacterial cell, a yeast cell, an insect cell or a mammalian cell, such as a human cell. A preferred recombinant cell is selected from a cell suitable for recombinant expression of the nanostructure of this invention.

Another aspect of this invention relates to a pharmaceutical composition comprising a nanostructure, a system, a composition, a substance, a vector, and/or a host cell as defined above, together with a pharmaceutically acceptable carrier and/or excipient.

The pharmaceutical composition of the invention shall be formulated to be compatible with its intended route of administration. In a particularly preferred embodiment, examples of routes of administration of the pharmaceutical and/or compound of this invention include intravenous, vaginal, oral, intranasal, intrathecal, intra-arterial, intradermal, subcutaneous, transdermal (topical), intracerebroventricular, intraparenchymal, intratumoral, transmucosal, rectal, bronchial, parenteral administration, and any other clinically/medically accepted method for administration of a pharmaceutical and/or a compound.

Yet another aspect of this invention, which can be combined with any of the other aspects or specific embodiments of this invention, relates to a method of preventing and/or treating a proliferative disease, such as cancer, an immunological disorder, a viral disease, such as a human immunodeficiency virus (HIV) infection, a blood clotting disorder, a metabolic disorder, an infectious disorder, and/or diabetes, the method comprising administering to a subject a therapeutically effective amount of a nanostructure, a system, a compound, a substance, and/or a pharmaceutical composition according to this invention.

To calculate the amount required to be a "therapeutically effective amount", the skilled person and/or the physician can use data obtained from cell culture assays and animal studies to formulate a range of dosage for use in humans. The dosage of such compounds and/or pharmaceutical compositions lies preferably within a range of circulating concentrations that include the ED50 with little or no toxicity. The dosage may vary within this range depending upon the dosage form employed and the route of administration utilized. For any compound used, the therapeutically effective dose can be estimated initially from cell culture assays. A dose may be formulated in animal models to achieve a circulating plasma concentration range that includes the IC50 (i.e., the concentration of the test compound which achieves a half-maximal inhibition of symptoms) as determined in cell culture. Such information can be used to more accurately determine useful doses in humans. The pharmaceutical compositions can be included in a container, pack, or dispenser, together with instructions for administration.

This invention further relates to a nanostructure, a system, a compound, a substance, and/or a pharmaceutical composition according to this invention, for use in the manufacture of a medicament for preventing and/or treating a proliferative disease, such as cancer, an immunological disorder, a viral disease, such as a human immunodeficiency virus (HIV) infection, a blood clotting disorder, a metabolic disorder, an infectious disorder, and/or diabetes.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure. In particularly preferred embodiments of the invention, the term "about" may refer to a deviation of the respective numeric value of a maximum of 20% of the numerical value, however more preferred is 15%, 10%, 5%, even more preferred is 4%, 3%, 2%, and most preferred is 1%. However, whenever a relative term, such as "about", "substantially" or "approximately" is used in this specification, such a term should also be construed to also include the exact term. That is, e.g., "substantially one" should be construed to also include "(exactly) one".

It is to be understood that application of the teachings of the present invention to a specific problem, and the inclusion of variations of the present invention or additional features thereto (such as further aspects and embodiments), will be within the capabilities of one having ordinary skill in the art in light of the teachings contained herein. Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:

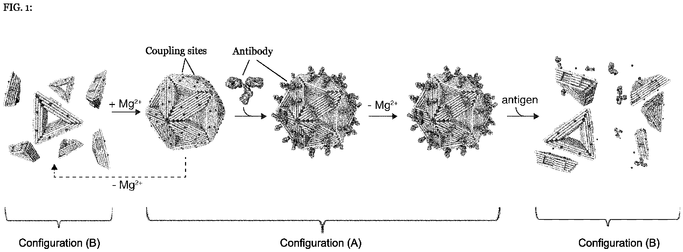

**Figure 1** shows IgG mediated stabilization and antigen triggered disassembly of icosahedral DNA origami shells. Antigens strategically mounted on the DNA-origami subunits ("entities", shown in a triangular shape) allow bivalent antibodies ("coupling molecules") to act as molecular bridges between different DNA-origami subunits ("entities", shown in a triangular shape) to stabilize subunit interactions at otherwise unfavourable conditions when bound to antigenic fragments. Shape complementary protrusions and recesses govern the self-assembly of icosahedral shells from 20 identical copies of a multi-layer DNA origami subunit. Self-assembly of the icosahedral shell requires the addition of Mg2+ ions to stabilize blunt end stacking contacts between the shape complementary features. Antibody-specific antigens are installed at symmetric locations on the DNA-origami subunit ("triangle") monomer surface. Shell assembly results in the positioning of the antigens at opposing sides of each triangle-triangle interface. Antigen-specific antibodies ("coupling molecules") bivalently bind to the shell-mounted antigens and stabilize the triangle-triangle interactions. By subsequently diluting the icosahedral shells to otherwise destabilizing Mg2+ ion concentrations the shells remain in a metastable, "spring-loaded" state. Controlled disassembly is triggered by the addition of auxiliary antigens ("competing antigens") that displace the stabilizing antibody from the shell, thereby inducing a conformational reconfiguration of the DNA-origami structure. Legend: Mg²⁺ = magnesium ion; 1 Triangle = 1 DNA-origami subunit ("entity"); "coupling site" = antigen fragment (preferably mounted onto DNA-origami subunits via staple strands); antibody = "coupling molecule".

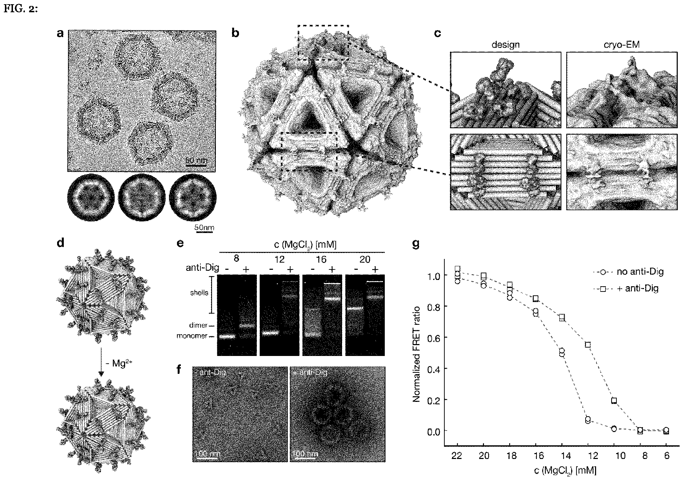

**Figure 2** shows structural characterization and evaluation of Mg2+ dependent IgG mediated shell stabilization. (a) Top: Exemplary Cryo-EM micrograph of icosahedral shells in free-standing ice with anti-Digoxigenin antibodies cross-linking the triangle-triangle interfaces at 25 mM MgCl2. Bottom: Two-dimensional class averages from different orientations. (b) Cryo-EM reconstruction of the assembled icosahedral shell with two anti-Digoxigenin antibodies bridging each triangle-triangle interface. (c) Zoom-in at a triangle-triangle interface crosslinked by two anti-Digoxigenin antibodies depicted in (left) a cylinder model with manually positioned IgG antibodies (PDB: 1IGT) and (right) Cryo-EM reconstruction. Notably, in addition to both Fab fragments, parts of the antibody's Fc region are reconstructed. (d) Schematic representation of antibody-mediated stabilization at decreased Mg2+ concentrations. (e) Laser-scanned fluorescence images of 0.75% agarose gels in 0.5X TBE buffer supplemented with 8, 12, 16 and 20 mM MgCl2. Assembled shells were incubated with and without anti-Digoxigenin antibodies at 25 mM MgCl2 and subsequently diluted to the respective MgCl2 concentrations and electrophorized on the corresponding gels. Legend: Monomer = single triangle monomers, dimer = dimers formed by 2 triangle subunits, shells = fully assembled icosahedral shells. (f) Negatively-staining TEM images of shells incubated without (left) or with (right) anti-Digoxigenin antibodies and diluted to 12 mM MgCl2. (g) Normalized FRET ratio of shells incubated without and with anti-Digoxigenin antibody and subsequently diluted to various final MgCl2 concentrations. Each side of the triangle subunit is functionalized with a Cy3 and Cy5 fluorophore at symmetric locations, resulting in energy transfer only when shells are assembled. The initial decrease in FRET ratio at MgCl2 concentrations between 22 to 14 mM is caused by swelling of the shells due to increasing electrostatic repulsion.

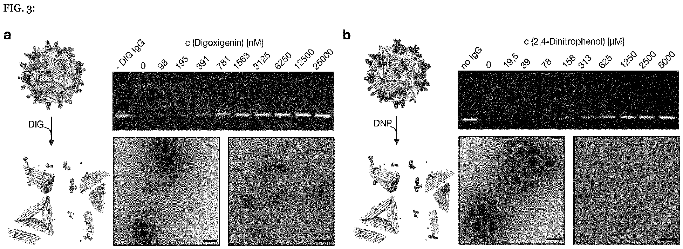

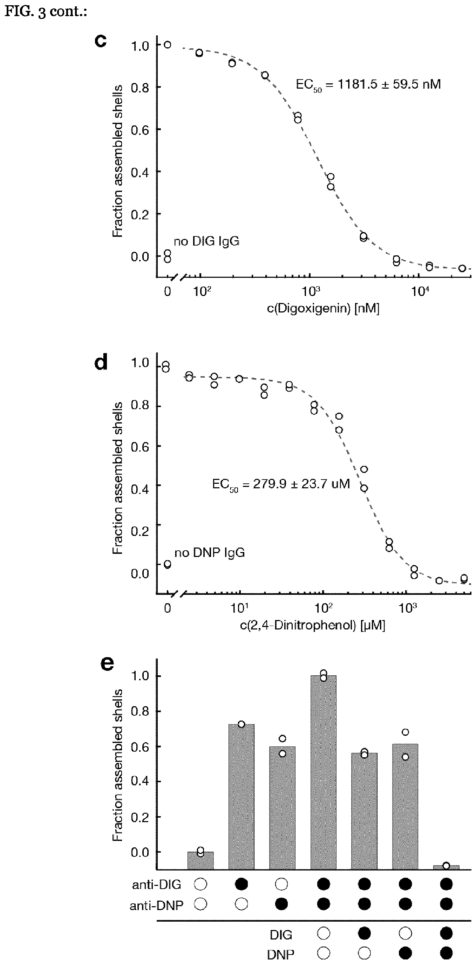

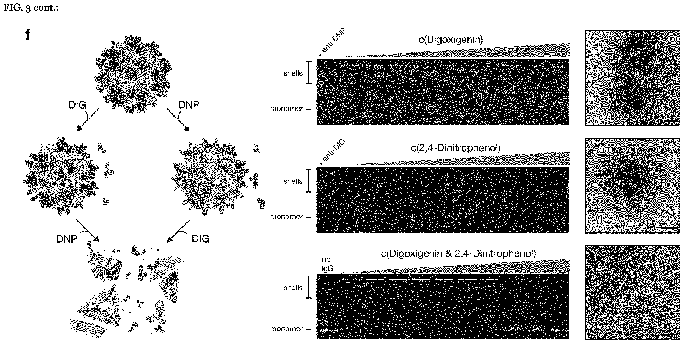

Figure 3 shows antigen-triggered disassembly of icosahedral shells. (a) By the addition of soluble Digoxigenin ligands that compete for binding with the shell-stabilizing anti-Digoxigenin antibody, the antibody is displaced from the spring-loaded shell, consequently triggering the disassembly of the shell at destabilizing Mg2+ ion concentrations. Laser-scanned fluorescence images of 0.75% agarose gels in 0.5X TBE buffer supplemented with 12 mM MgCl2. Assembled shells equipped with anti-Digoxigenin antibodies were incubated with increasing concentrations of Digoxigenin. Anti-Digoxigenin stabilized shells disassemble at increasing concentrations of soluble Digoxigenin antigens. Negative-staining TEM images of anti-Digoxigenin stabilized shells in absence (left) and presence (right) of 25 µM soluble Digoxigenin. (b) Similar as in (a) but with anti-Dinitrophenol stabilized shells and addition of soluble 2,4-Dinitrophenol ligand. (c) Normalized FRET ratio of shells stabilized by anti-Digoxigenin at increasing concentrations of soluble Digoxigenin ligands. (d) Normalized FRET ratio of shells stabilized by anti-Dinitrophenol at increasing concentrations of soluble 2,4-Dinitrophenol. The dashed lines represent non-linear least squares optimization of the Hill equation to the experimental data. (e) Left: AND-gate logic gated shell disassembly is realized by installing both Digoxigenin and 2,4-Dinitrophenol ligands and the respective antibodies on the triangle-triangle interfaces. Disassembly of the AND-gate shell consequently requires the presence of both soluble Digoxigenin and 2,4-Dinitrophenol. Right: Laser-scanned fluorescence images of 0.75% agarose gels in 0.5X TBE buffer supplemented with 12 mM MgCl2. Assembled AND-gate shells equipped with anti-Digoxigenin and anti-Dinitrophenol antibodies were incubated with increasing concentrations of top: Digoxigenin, middle: 2,4-Dinitrophenol and bottom: Digoxigenin and 2,4-Dinitrophenol. Negative-staining TEM images of shells equipped with anti-Digoxigenin and anti-Dinitrophenol incubated with top: 25 µM Digoxigenin, middle: 5 mM 2,4-Dinitrophenol and bottom: 25 µM Digoxigenin and 5 mM 2,4-Dinitrophenol. (f) Normalized FRET ratio of AND-gate shells stabilized by combinations of anti-Digoxigenin and anti-Dinitrophenol, and incubated with combinations of soluble Digoxigenin (25 µM) and 2,4-Dinitrophenol (5mM).

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: IgG mediated stabilization and antigen triggered disassembly of icosahedral DNA origami shells

This invention is based on a generic strategy for antigen-triggered logic gated reconfiguration of DNA origami nanoparticles. The design principle of the technique of this invention is inherently modular and can be applied to any dynamic DNA origami system that can adopt two or more configurational states.

Fig. 1 depicts a component used in some embodiments of the present technology to illustrate notations used in the present specification. More particularly, Fig. 1 depicts a nanostructure that can assume different configurations (A) and (B), which may also be referred to as a closed configuration (A) and an open configuration (B). A three-dimensional nanostructure, as depicted in Fig. 1, may be realized using DNA origami, i.e. by combining scaffolding strands and staple stands to form the required portions and the overall device. Such designs may, for example, be performed using software such as caDNAno. That is, a nanostructure comprising multiple entities may in some embodiments be made out of one scaffolding strand, whereas in other embodiments entities of a nanostructure may be constructed utilizing a plurality of scaffolding strands.

The nanostructure is preferably configured to assume the first configuration (A) when each, or at least a subset of, coupling sites of a coupling site set is coupled by its respective coupling molecule, and to assume the second configuration (B) when none, or a reduced amount of a subset of, the coupling sites is coupled by its respective coupling molecule.

In the embodiment depicted in Fig. 1, the coupling sites are of the same configuration, i.e., they are configured to couple to coupling molecules of the same design, and the nanostructure is of a bivalent nature, i.e., a nanostructure having one type of coupling sites. However, as already mentioned above, this is not necessary. In some embodiments of the present invention, the coupling sites may bind to distinct coupling molecules. The latter refers to a multivalent nanostructure, that is a structure with a plurality of different coupling sites.

The proposed mechanism depicted in Fig. 1 is based on two design principles. First, the inventors used the highly conserved structure of IgG antibodies, which bivalently display two identical antigen binding sites. Exploiting this bivalent character, antibodies are used as molecular bridges to lock the DNA origami nanoparticle in a pre-defined configuration, governed by strategically located antigen(-fragments) on the DNA origami subunits. Second, antigen-triggered activation or reconfiguration of the "spring-loaded" nanoparticle is initiated by the presence of soluble antigens that displace the "antibody bridges" from the nanoparticle.

In the example of Fig. 1, the configuration (A) is a closed configuration (such as a "cage"-like structure, e.g. a capsid-like structure assembled from, in this particular embodiment, 20 identical DNA-origami subunits (triangles) are forming icosahedral DNA origami shells). Configuration (B) is an open configuration, in which the DNA origami subunits (triangles) are not coupled via the antibody-bridges. Therefore, single triangles are visible in negative-staining TEM imagines of configuration (B).

As a proof-of-principle, Fig. 1 shows the antibody mediated spring loading and subsequent antigen triggered disassembly of a T=i capsid-like structure (icosahedral DNA origami shells), self-assembled from 20 identical multi-layer DNA origami triangles (Fig. 1). To govern antibody binding, "coupling sites" (antigen fragments, e.g. digoxigenin ligands) are introduced at symmetrical locations on each triangle side via chemically modified staple oligonucleotides. After self-assembly of the icosahedral shell (T=i capsid), governed by the activation of shape-complementary recesses and protrusions on the triangle interfaces at shell favoring buffer conditions ([Mg2+] > 20 mM), anti-digoxigenin antibodies are added to crosslink the individual triangle subunits. Subsequent decrease of the Mg2+ concentration to deactivate the stacking contacts results in spring loading of the capsid assembly. Finally, addition of auxiliary antigens ("competing antigens", e.h. soluble digoxigenin antigens) trigger capsid disassembly by competitive reactions that remove the antibody from the DNA-origami mounted antigens (Fig. 1). Antigen-triggered disassembly of the icosahedral shell occurs at a particular antigen threshold (EC50 ∼ 1 µM), which is dependent on the number of mounted antibodies per subunit interface. This concept can be further explored by installing specific combinations of antibodies, creating sophisticated molecular logic operations or tailoring dose-response curves.

While the embodiment of Fig. 1 is based on a particle self-assembled from multiple components as proof-of-principle, the same concept can be applied to actuate unimolecular objects such as box like structures or switches, with applications including but not limited to molecular diagnostics, logic-gated therapeutics or drug delivery vehicles. Finally, given the highly conserved bivalent nature of, e.g., IgG antibodies and the ease of generating antibodies against virtually any molecular target, this invention could be employed for identifying a wide variety of antigens, small molecules, peptides and proteins.

In contrast to current modules that interface DNA nanostructures with antigen-antibody recognition elements, all recognition elements of a nanostructure as depicted in Fig. 1, are integrated on the nanostructure itself, rather than relying on diffusive interactions of multiple components. This makes the inventors' concept insensitive to dilution effects, which is a crucial requirement for potential future biomedical applications. Importantly, the inventors' design principle is not limited to multi-component systems whose self-assembly is mediated by shape complementary stacking contacts. Other strategies like temperature or strand displacement dependent complementary sticky ends, pH-dependent triplexes or i-motifs could be envisioned to direct self-assembly of the nanostructures of this invention, and subsequent spring loading of the structure by antibody reinforced interfaces, which shall also be within the scope of this invention.

### Example 2: structural characterization of antigen-triggered disassembly of icosahedral shells

To structurally characterize and evaluate Mg2+ dependent IgG mediated shell stabilization, the inventors performed Cryo-EM and Laser-scanning fluorescence microscopy. Exemplary Cryo-EM micrographs of icosahedral shells in free-standing ice with anti-Digoxigenin antibodies cross-linking the triangle-triangle interfaces at 25 mM MgCl2 are shown in Fig. 2 (a), Top. Cryo-EM reconstruction of the assembled icosahedral shell with two anti-Digoxigenin antibodies bridging each triangle-triangle interface are shown in Fig. 2 (a), bottom.

Then, the inventors performed Laser-scanning microscopy. Fig. 2 e) shows Laser-scanned fluorescence images of 0.75% agarose gels in 0.5XTBE buffer supplemented with 8, 12, 16 and 20 mM MgCl2. Assembled shells were incubated with and without anti-Digoxigenin antibodies at 25 mM MgCl2 and subsequently diluted to the respective MgCl2 concentrations and electrophorized on the corresponding gels. The inventors further used TEM to characterize configuration (A) and configuration (B). Negatively-staining TEM images of shells incubated without (left) or with (right) anti-Digoxigenin antibodies and diluted to 12 mM MgCl2 are shown in Fig. 2 (f), and the normalized FRET ratio of shells incubated without and with anti-Digoxigenin antibody and subsequently diluted to various final MgCl2 concentrations is shown in Fig. 2 (e). Each side of the triangle subunit is functionalized with a Cy3 and Cy5 fluorophore at symmetric locations, resulting in energy transfer only when shells are assembled. The initial decrease in FRET ratio at MgCl2 concentrations between 22 to 14 mM is caused by swelling of the shells due to increasing electrostatic repulsion. To further characterize antigen-triggered disassembly of icosahedral shells, the inventors used Laser-scanning microscopy to demonstrate antigen-triggered disassembly of icosahedral shells. Fig. 3 (a) shows that the addition of soluble Digoxigenin ligands that compete for binding with the shell-stabilizing anti-Digoxigenin antibody, the antibody is displaced from the spring-loaded shell, consequently triggering the disassembly of the shell at destabilizing Mg2+ ion concentrations. Assembled shells equipped with anti-Digoxigenin antibodies were incubated with increasing concentrations of Digoxigenin to demonstrate that anti-Digoxigenin stabilized shells (25 µM soluble Digoxigenin) disassemble at increasing concentrations of soluble Digoxigenin antigens, as shown in Fig. 3 (a). Fig. 3 (b) shows anti-Dinitrophenol stabilized shells disassemble at increasing concentrations of soluble 2,4-Dinitrophenol ligands.

Fig. 3 (f) shows in the left part AND-gate logic gated shell disassembly is realized by installing both Digoxigenin and 2,4-Dinitrophenol ligands and the respective antibodies on the triangle-triangle interfaces. Disassembly of the AND-gate shell consequently requires the presence of both soluble Digoxigenin and 2,4-Dinitrophenol. Fig. 3 (f) shows in the right part Laser-scanned fluorescence images of 0.75% agarose gels in 0.5X TBE buffer supplemented with 12 mM MgCl2. Assembled AND-gate shells equipped with anti-Digoxigenin and anti-Dinitrophenol antibodies were incubated with increasing concentrations of top: Digoxigenin, middle: 2,4-Dinitrophenol and bottom: Digoxigenin and 2,4-Dinitrophenol. Negative-staining TEM images of shells equipped with anti-Digoxigenin and anti-Dinitrophenol incubated with top: 25 µM Digoxigenin, middle: 5 mM 2,4-Dinitrophenol and bottom: 25 µM Digoxigenin and 5 mM 2,4-Dinitrophenol.

While in the above a preferred embodiment has been described with reference to the accompanying drawing, the skilled person will understand that this embodiment was provided for illustrative purpose only and should by no means be construed to limit the scope of the present invention, which is defined by the claims.

## Claims

1. A nanostructure comprising at least two coupling sites, wherein said at least two coupling sites may bind to at least one coupling molecule, and wherein said at least two coupling sites are configured to be a coupling site set, optionally wherein one coupling site set is configured to bind to one coupling molecule.

2. The nanostructure according to claim 1, wherein the nanostructure comprises at least one first entity and at least one second entity, wherein each of the at least one first entity and the at least one second entity comprises at least one coupling site of at least one coupling site set, preferably wherein the at least one coupling site of the at least one first entity and the at least one coupling site of the at least one second entity are located on symmetrical locations of the at least one first and of the at least one second entity, optionally wherein the at least one coupling molecule is configured to couple the at least one first entity of the nanostructure to the at least one second entity of the nanostructure when the at least one coupling molecule is binding to the at least one coupling site set.

3. The nanostructure according to claim 1 or 2, wherein the nanostructure comprises a plurality of the at least one first entity and of the at least one second entity, such as at least two, three, four, five, six, seven, eight, nine, ten, eleven, twelve, thirteen, fourteen, fifteen, sixteen, seventeen, eighteen, nineteen, twenty, twenty one, twenty two, twenty three, twenty four, twenty five, twenty six, twenty seven, twenty eight, twenty nine, thirty, or any number of the at least one first entity, and at least one, two, three, four, five, six, seven, eight, nine, ten, eleven, twelve, thirteen, fourteen, fifteen, sixteen, seventeen, eighteen, nineteen, twenty, twenty one, twenty two, twenty three, twenty four, twenty five, twenty six, twenty seven, twenty eight, twenty nine, thirty, or any number of the at least one second entity, optionally wherein the nanostructure comprises a plurality of coupling site sets, preferably at least two, three, four, five, six, seven, eight, nine, ten, eleven, twelve, thirteen, fourteen, fifteen, sixteen, seventeen, eighteen, nineteen, twenty, twenty one, twenty two, twenty three, twenty four, twenty five, twenty six, twenty seven, twenty eight, twenty nine, thirty, or any number of coupling site sets, preferably wherein each coupling site set consists of two coupling sites.

4. The nanostructure according to any of the preceding claims, wherein the nanostructure is configured to assume at least one first configuration (A), and at least one second configuration (B), optionally at least one third configuration (C), at least one fourth configuration (D), or any other number of configurations.

5. The nanostructure according to claim 4, wherein the nanostructure is configured to assume the at least one first configuration (A) when at least one coupling molecule is binding to at least one coupling site set, and/or wherein the nanostructure is configured to assume the at least one second configuration (B) when at least one coupling molecule is not binding to at least one coupling site set, optionally wherein the nanostructure is configured to assume the at least one first configuration (A) when a little or no amount of at least one competing molecule is present, and/or wherein the nanostructure is configured to assume the at least one second configuration (B) when a higher amount of at least one competing molecule is present.

6. The nanostructure according to claim 4 or 5, wherein the nanostructure is configured to assume the at least one first configuration (A) in a first solution, wherein the first solution preferably comprises a higher concentration of magnesium ions compared to a second solution, and/or wherein the nanostructure is configured to assume the at least one second configuration (B) in the second solution, wherein the second solution preferably comprises a lower concentration of magnesium ions compared to the first solution, optionally wherein the magnesium ion concentration of the first solution is higher than 20 mM, and/or the magnesium ion concentration of the second solution is lower than 20 mM.

7. The nanostructure according to any of the preceding claims, wherein the nanostructure comprises at least one molecule selected from the group consisting of a nucleic acid, such as a DNA, an RNA, a DNA aptamer, an RNA aptamer, or a peptide nucleic acid (PNA), a polypeptide, a peptide, a glycoprotein, a peptidomimetic, and an antigen binding construct, such as an antibody, an antibody-like molecule, an antigen binding derivative, or an antigen binding fragment thereof.

8. The nanostructure according to any of the preceding claims, wherein the nanostructure is at least partially formed by a DNA origami structure,
wherein the DNA origami structure comprises at least one scaffolding strand; wherein the DNA origami structure further comprises a plurality of single-stranded oligonucleotide staple strands;
wherein each staple strand is at least partially complementary to at least one scaffolding strand; and
wherein each of the staple strands is configured to bind to at least one of the at least one scaffolding strands, wherein the at least one scaffolding strand is folded and/or arranged such that the desired nanostructure is formed.

9. The nanostructure according to any of the preceding claims, wherein the at least one coupling site is a molecule, preferably wherein the at least one coupling site, and/or the at least one coupling molecule is a molecule selected from the group consisting of an antigen, an antigenic fragment, an antigenic mimetic, an antigen binding construct, such as an antibody, an antibody-like molecule, an antibody mimetic, an antigen binding derivative, or an antigen binding fragment, a DNA strand, a biotin molecule, a streptavidin molecule, a maleimide-thiol chemistry molecule, a click chemistry molecule, a SNAP-tag protein, and a CLIP-tag molecule, preferably wherein the coupling molecule is an antigen binding construct, such as an antibody, an antibody-like molecule, an antibody mimetic, an antigen binding derivative, or an antigen binding fragment thereof, and wherein the coupling site is an antigen, an antigenic fragment, and/or an antigenic mimetic.

10. The nanostructure according to any of the preceding claims, wherein the coupling molecule is an IgG antibody, an IgG antibody fragment, an IgG antibody mimetic, an IgM antibody, an IgM antibody fragment, an IgM antibody mimetic, an IgD antibody, an IgD antibody fragment, an IgD antibody mimetic, an IgA antibody, an IgA antibody fragment, an IgA antibody mimetic, an IgE antibody, an IgE antibody fragment, or an IgE antibody mimetic, preferably wherein said antibody, antibody fragment, or antibody mimetic comprises two identical antigen binding sites, more preferably wherein said antigen binding sites bind to said at least two coupling sites of a coupling site set.

11. The nanostructure according to any of the preceding claims, wherein the nanostructure comprises a maximum length, and wherein the maximum length is smaller than 1000 nm, preferably smaller than 500 nm, such as 100 nm.

12. A system comprising the nanostructure according to any of the preceding claims, wherein the system comprises
(i) at least one nanostructure comprising at least two coupling sites, wherein said at least two coupling sites may bind to at least one coupling molecule, and wherein said at least two coupling sites are configured to be a coupling site set, and
(ii) at least one coupling molecule.

13. The system according to claim 12, wherein the system comprises
at least one nanostructure comprising at least one first entity and at least one second entity, wherein each of the at least one first entity and the at least one second entity comprises at least one coupling site of at least one coupling site set, preferably wherein the at least one coupling site on the at least one first entity and the at least one coupling site on the at least one second entity are located on symmetrical locations of the entities, and
at least one coupling molecule, preferably a plurality of coupling molecules,
wherein the at least one coupling molecule is configured to couple the at least one first entity of the nanostructure to the at least one second entity of the nanostructure when the at least one coupling molecule is binding to the at least one coupling site set.

14. A method for linking an identification of at least one molecule to a conformational reconfiguration of a nanostructure, wherein the method comprises:
(i) providing a nanostructure according to any one of the claims 1 to 11;
(ii) providing at least one coupling molecule,
wherein the nanostructure is assuming at least one first configuration (A) when at least one coupling molecule as provided in (ii) is binding to at least one coupling site set of the nanostructure provided in (i), and
(iii) identifying the at least one molecule,
wherein said at least one molecule is preferably a competing molecule,
wherein the nanostructure is assuming the at least one second configuration (B) upon identifying the at least one molecule,
thereby enabling linking the identification of at least one molecule to a conformational reconfiguration of the nanostructure,
optionally wherein the nanostructure encloses a molecule or a second nanostructure in the at least one first configuration (A), preferably wherein the molecule is selected from a therapeutic molecule and/or a cargo molecule,
further optionally wherein the molecule or second nanostructure, preferably the molecule selected from a therapeutic molecule and/or a cargo molecule, is released and/or activated upon conformational reconfiguration of the nanostructure when the nanostructure is assuming the at least one second configuration (B).

15. A composition comprising at least one or a plurality of nanostructures according to any one of the claims 1 to 11, or a system according to claim 12 or 13, for use as a medicament, and/or for use in a diagnostic method.
